# EUROPEAN PATENT APPLICATION

(11) **EP 1 543 794 A1**
(43) Date of publication of application: **22.06.2005**
(21) Application number: 03028991.2
(22) Date of filing: 17.12.2003
(51) Int. Cl.: A61C 5/06, A61C 1/16

(54) **A system for storing and dispensing a dental material having gel consistency**

(71) Applicant: DENTSPLY DETREY GmbH, 78467 Konstanz (DE)
(72) Inventor: Grützner, Andreas E., Dr., 78479 Reichenau (DE)
(74) Representative: Hartz, Nikolai F., Dr.

(57) **Abstract**

A system for dispensing a dental material having gel consistency, said system comprising (a) a multiple-use dispensing device with (a-1) a tubular body having a distal end and a proximal end, said body containing said dental material in an amount sufficient for application to more than one patient, (a-2) a dispensing opening provided at the distal end of said body, (a-3) a movable piston, and (a-4) a plunger connected to said piston for dispensing said dental material through said dispensing opening; (b) a plug for closing said dispensing opening; (c) a single-use tubular dispensing tip having a proximal end and a distal end, said dispensing tip being connectable in a fluid-flow communication to said dispensing opening after removal of said plug, and said distal end having a dispensing orifice for dispensing said dental material into an oral environment; and (d) a single-use elastic envelope adapted for shielding said dispensing device or at least a distal portion thereof from contamination by the oral environment.

## Description

### Technical field and background of the invention

The present invention relates to a system for storing and dispensing a dental material having gel consistency.

There are different types of systems which have conventionally been used for storing dental materials and dispensing or applying them to tissue of the oral environment of a patient. One type of these systems comprises a multiple-use ejector holder and capsule-like cartridges for dough-like high-viscous dental filling materials as disclosed in EP 0 145 922 A2 and EP 0 744 161 A1. Such systems have the advantage that the ejector holder may be disinfected by autoclaving, and thus it may be used repeatedly. If used appropriately, the ejector holder is disinfected before using it with a patient. A cartridge usable with such ejector holders contains dental material in an amount sufficient in average for application to one patient. If used appropriately, at least one cartridge is needed for one patient. Namely, such a cartridge is used only for one patient and it is disposed after use. Hence, cross-contamination and cross-infections of patients are generally avoided. However, in case that only a small amount of dental material is needed for one patient, a large amount of dental material is wasted. Further, in case that a large amount of dental material is needed for one patient, a further cartridge may be necessary, and the amount of dental material remaining in the further cartridge after use may be also a large amount of dental material which is wasted. This drawback has led to the practice of some users to reuse cartridges containing still large amounts of dental material. Since such cartridges cannot be disinfected by autoclaving a thorough disinfection is not possible. Hence, cross-contamination and cross-infections of patients treated with dental material from the same cartridge are possible. On the other hand, another type of systems comprises a dispensing device containing dental material sufficient for more than one patient, e.g. the system of an etching gel such as the DeTrey Conditioner 36. This system comprises a multiple-use syringe containing an etching gel and a disposable cannula for single-use. However, even after disposing the single-use cannula after treatment of a patient, cross-contaminations and cross-infections of different patients treated with the same dispensing device may occur. Namely, the dispensing device containing the dental material cannot be disinfected by autoclaving. Further, not only the single-use cannula is contacted with saliva and other body fluids of a patient during use but also the fingers of the user and the dispensing device itself are contacted with saliva and other body fluids of the treated patients.

Hence, the object of the present invention is to overcome the drawbacks of the systems according to the prior art. Particularly, it is the object of the present invention to provide a system for dispensing a dental material from a multiple-use dispensing device containing dental material in an amount sufficient for more than one patient, whereby cross-contaminations and cross-infections of patients during use of the multiple-use dispensing device is prevented.

### Summary of the present invention

The present invention provides a system for dispensing a dental material having gel consistency, said system comprising:
(a) a multiple-use dispensing device with
   (a-1) a tubular body having a distal end and a proximal end, said body containing said dental material in an amount sufficient for application to more than one patient,
   (a-2) a dispensing opening provided at the distal end of said body,
   (a-3) a movable piston, and
   (a-4) a plunger connected to said piston for dispensing said dental material through said dispensing opening;
(b) a plug for closing said dispensing opening;
(c) a single-use tubular dispensing tip having a proximal end and a distal end, said dispensing tip being connectable in a fluid-flow communication to said dispensing opening after removal of said plug, and said distal end having a dispensing orifice for dispensing said dental material into an oral environment; and
(d) a single-use elastic envelope adapted for shielding said dispensing device or at least a distal portion thereof from contamination by the oral environment.

In a preferred embodiment of the present invention the elastic envelope of the system for dispensing a dental material is connected to said dispensing tip. Hence, a user cannot assemble the dispensing tip onto the multiple-use dispensing device without the elastic envelope. An inappropriate use is thus essentially prevented, even if the user is in a hurry or forgets to assemble the elastic envelope.

In a particular preferred embodiment of the present invention, the elastic envelope of the system for dispensing a dental material is a flexible tubular sheath with a first and second end, whereby said first end is provided at the proximal end of said tubular dispensing tip. This embodiment has the advantage that the elastic envelope is accommodated to the shape of the tubular body of the multiple-use dispensing device. Hence, the user has good sight when applying dental material to an application site within the mouth of a patient. Further, the handling of such a system is advantageously improved.

According to a further embodiment of the present invention, the second end of the sheath of the elastic envelope of the system for dispensing a dental material can be tightened, preferably by a cord or ribbon. In a still further preferred embodiment such cord or ribbon is provided at the elastic envelope. According to another embodiment of the present invention a tightening means, such as a clamping device, may be provided at the tubular body of the multiple-use dispensing device. Such clamping device can be for example a sleeve which may be slid over the second end of the flexible tubular sheath of the elastic envelope.

According to a particularly preferred embodiment of the present invention, the elastic envelope of the system for dispensing a dental material is folded up or rolled up prior to use. This embodiment has the advantage that the system is ready to use in a very short time, since the step of assembling the elastic envelope on the tubular body or on the dispensing tip is eliminated. Hence, expensive time is saved at the chair-side application of the dental material by the user.

In a further preferred embodiment of the system for dispensing a dental material according to the present invention, the contact surfaces of the dispensing tip and the tubular body of the multiple-use dispensing device are adapted for a snug fitting. In this way creeping of dental material into a space between the dispensing tip and the tubular body is essentially avoided.

According to a further preferred embodiment of the system of the present invention, the tubular dispensing tip can be bent for accommodating the oral situation.

In a preferred embodiment of the present invention, the tubular dispensing tip is adapted for engaging with said dispensing device, preferably the distal end of the tubular body of the dispensing device, with a positive lock. According to this embodiment loosening of the tubular dispensing tip and leakage of the system during use is avoided. The positive lock may be achieved by a thread, a bayonet locking, or a snapping connection between the tubular dispensing tip and the dispensing device.

### Brief description of the drawings

Fig. 1A is a sectional side view of a disassembled system for dispensing a dental material having gel consistency according to the present invention.
Fig. 1B is a sectional side view of the assembled system according to Fig. 1A.
Fig. 2 is a sectional side view of an assembled system for dispensing a dental material having gel consistency according to a further embodiment of the present invention.
Fig. 3 is a top view of the elastic envelope of Fig. 2.
Fig. 4 is a top view of an elastic envelope of a further embodiment of the present invention.
Fig. 5A is a side view of a disassembled system for dispensing a dental material having gel consistency according to a further embodiment of the present invention.
Fig. 5B is a side view of the assembled system according to Fig. 5A.
Fig. 6A is a side view of a further system for dispensing a dental material having gel consistency according to the present invention.
Fig. 6B is a rear view of the system illustrated in Fig. 6A.
Fig. 6C is a side view of the system illustrated in Fig. 6A and 6B, whereby the second end of the tubular elastic envelope is tightened.
Fig. 7A is a sectional side view of a further embodiment of a disassembled system according to the present invention.
Fig. 7B is a sectional side view of the assembled system of Fig. 7A.

### Detailed description of the preferred embodiment

The present invention is now described in further detail with reference to the accompanying drawings. Corresponding or equivalent elements of the system are designated with the same reference signs in the drawings.

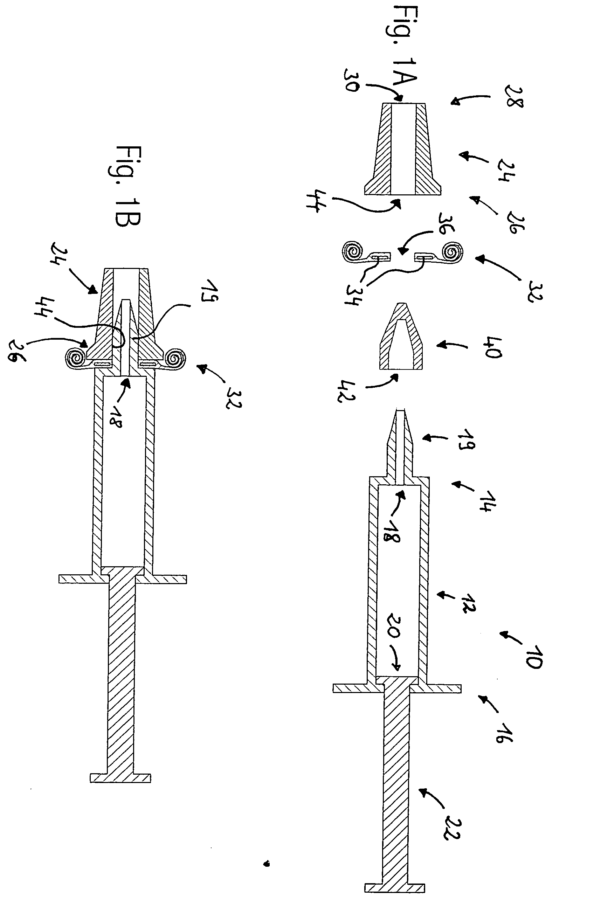

Fig. 1 shows a system for dispensing a dental material having gel consistency according to the present invention. It comprises the multiple-use dispensing device 10 with the tubular body 12 having the distal end 14 and the proximal end 16. The tubular body 12 is adapted for containing a dental material (not shown) in an amount sufficient for application to more than one patient. Further, the tubular body 12 has a dispensing opening 18 provided at the distal end 14. The dispensing opening 18 is running into the tube 19 protruding from the distal end 14. The tube 19 is a male component adapted for receiving the plug 40 having the female connection section 42. During use of the system the tube 19 receives after removal of the plug 40 the dispensing tube 24 having the female connection section 44. Further, the multiple-use dispensing device 10 comprises the movable piston 20 and the plunger 22 connected to said piston for dispensing dental material through the dispensing opening 18. Dental material sufficient for more than one patient may be contained in the tubular body 12 for storing before and between application of dental material to different patients. The dispensing opening 18 may also be an aperture provided at the distal end of the tubular body 18. In this case a corresponding plug has a male connection section for insertion into the aperture. Further, the single-use tubular dispensing tip 24 has the proximal end 26 and the distal end 28. The dispensing tip 24 is connectable in a fluid-flow communication to the dispensing opening 18 after removal of the plug 40 from the dispensing opening 18. The distal end 28 of the tubular dispensing tip 24 has the dispensing orifice 30 for dispensing dental material into an oral environment and for applying the dental material directly onto an application site. The single-use elastic envelope 32 having the aperture or opening 36 is rolled up. It contains the ring 34 adapted for facilitating the assembly of the elastic envelope onto the tube 19. The elastic envelope is adapted for shielding the dispensing device 10 or at least a distal portion thereof from contamination by the oral environment, preferably when it is rolled down the tubular body 12 of the dispensing device. The aperture or opening 36 in the elastic envelope enables a flow of dental material from the multiple-use dispensing device into the single-use tubular dispensing tip 24.

Fig. 1B shows the system of Fig 1A, whereby the elastic envelope 32 is mounted on the tube 19 of the dispensing opening 18. Further, the tubular dispensing tip 24 having the female connection section 44 at the proximal end 26 is mounted on the tube 19 of the dispensing opening 18. Advantageously, the elastic envelope 32 seated between the tubular dispensing tip 24 and the dispensing opening 18 acts as a seal. According to a further embodiment of the present invention the proximal end of the single-use tubular dispensing tip 24 has a recess accommodated for receiving the ring 34 which may be provided in the elastic envelope around the opening 36 of the elastic envelope.

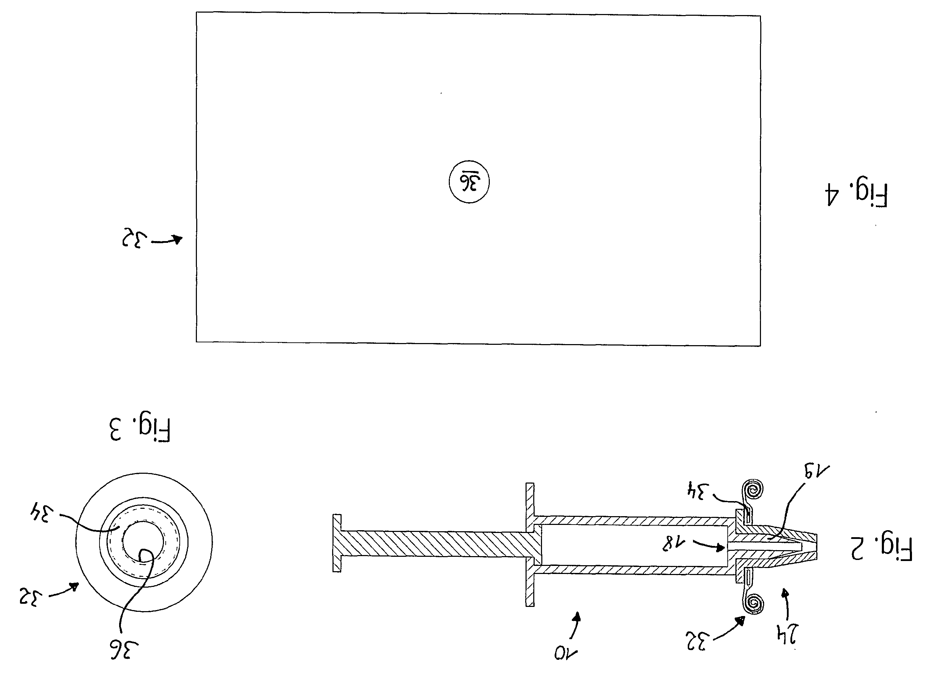

Fig. 2 shows another assembled embodiment of the system of the present invention. The tubular dispensing tip 24 is mounted on the tube 19 of the dispensing opening 18 of the dispensing device 10. The elastic envelope 32 is mounted as a rolled-up tubular sheath on the tubular dispensing tip 24. In a particularly preferred embodiment of the invention the elastic envelope is firmly connected to the tubular dispensing tip 24. Such a connection has the advantage that the tubular dispensing tip cannot be used without the elastic envelope.

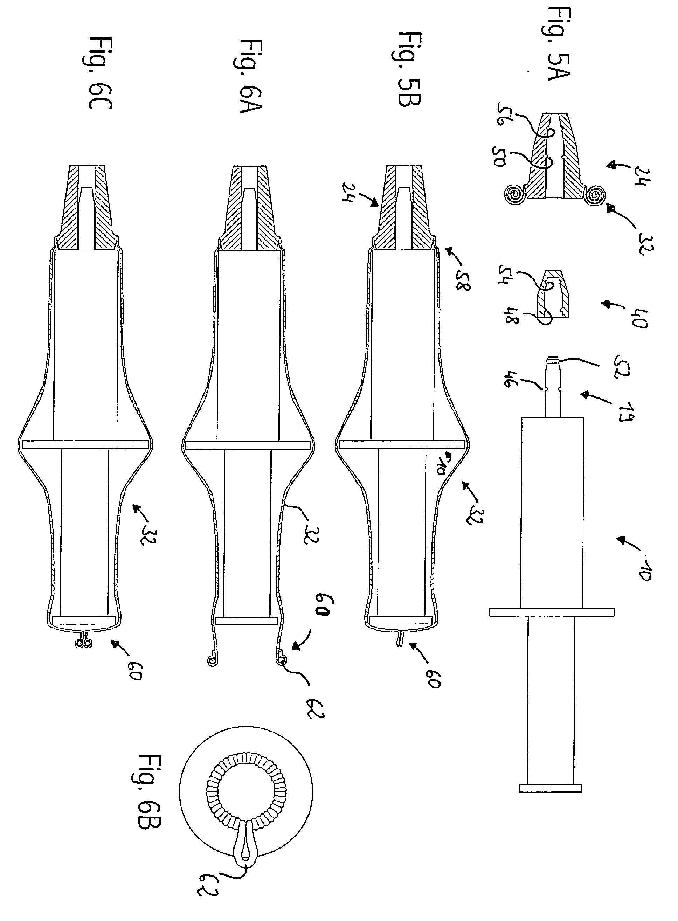

Fig. 3 shows the elastic envelope 32 of Fig. 2, which has the opening 36 for enabling the flow of dental material from the dispensing device through said envelope into a tubular dispensing tip. The elastic envelope is rolled up, and it contains a ring 34 for facilitating the placement of the elastic envelope over the tube 19 and/or the tubular dispensing tip 24 as shown in Fig. 1 B and Fig. 2, respectively.

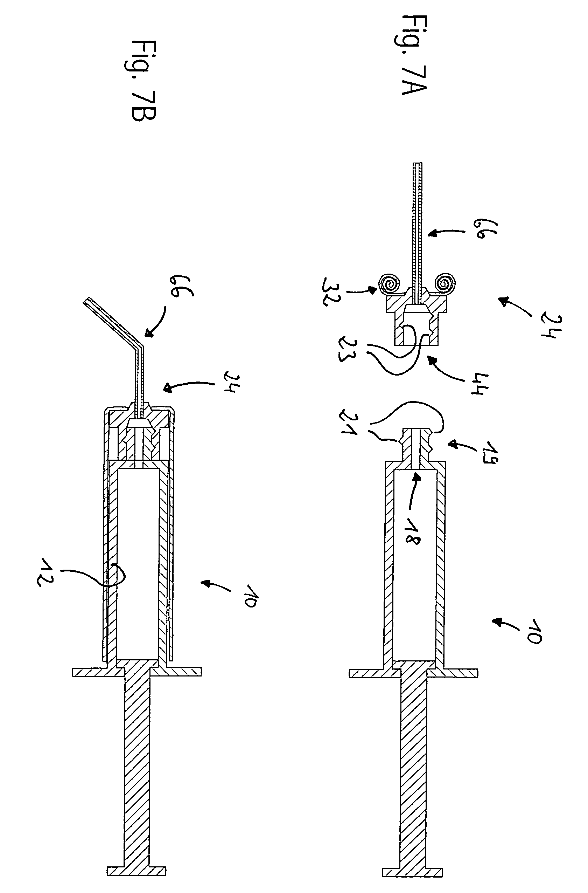

Fig. 4 shows an elastic envelope 32 in the form of a four-cornered sheath having the circular aperture 36 enabling the flow of dental material from the dispensing device through said envelope. The elastic envelope 32 may be placed on a dispensing opening 18 provided at the distal end of the body of a multiple-use dispensing device 10 as shown e.g. in Fig. 1A, 1 B or 2.

Fig. 5A shows a further embodiment of the system of the present invention. The dispensing opening of the dispensing device 10 runs into the tube 19 provided at the distal end of the tubular body of the dispensing device 10. The tube 19 has on its outer surface a recess 46 in the form of a groove adapted for a snapping connection with a corresponding protrusion in the form of an annular ring 48 on the inner surface of the female connection section of the plug 40 or a corresponding protrusion in the form of an annular ring 50 on the inner surface of the female connection section of the tubular dispensing tip 24. Further, the tube 19 has on its outer surface a protrusion 52 in the form of an annular ring adapted for a snapping connection with a corresponding recess in the form of a groove 54 on the inner surface of the female connection section of the plug 40 or a corresponding recess in the form of a groove 56 on the inner surface of the female connection section of the tubular dispensing tip 24. The corresponding recesses and protrusions provide a snapping connection between the dispensing device 10 and the plug 40 or the single-use tubular dispensing tip 24, respectively. Alternatively, the distal end of the multiple-use dispensing device may be connected to a plug or a single-use tubular dispensing tip by means of corresponding threads or a bayonet locking. The rolled up elastic envelope 32 is connected to the proximal end of the tubular dispensing tip 24.

Fig. 5B shows the system of Fig. 5A, whereby the elastic envelope 32 is rolled down the dispensing device 10. It is a flexible tubular sheath with the first end 58 and the second end 60, whereby the first end 58 is provided at the proximal end of the tubular dispensing tip 24. The tubular elastic sheath covers the whole dispensing device 10. The second end 60 of the sheath is closed. It can be tightened or clamped together, preferably by a cord or ribbon. Alternatively, it may be simply held together by the user holding the dispensing device 10 covered by the elastic envelope 32. According to a further embodiment of the invention the second end 60 may be glued together.

Fig. 6A shows a system similar to Fig. 5B, however, the elastic envelope 32 comprises at the second end 60 the cord 62. The second end 60 is already constricted to a certain degree by the use of the cord 62. Fig. 6B shows the rear view of the system shown in Fig. 6A. Fig. 6C shows the system of Fig. 6A and 6B, whereby the second end 60 is closed since the cord 62 constricts the second end 60 of the elastic envelope 32.

Fig. 7A shows a multiple-use dispensing device 10 having the dispensing opening 18 running into the tube 19 having the thread 21. The tube 19 with thread 21 is designed for being closed by a plug (not shown) having a corresponding thread and for being connected to the tubular dispensing tip 24 having a rolled up elastic envelope 32 connected thereto. The tubular dispensing tip has at its proximal end the female connection section 44 having the thread 23 corresponding to the thread 21 of the tube 19. Furthermore, the tubular dispensing tip 24 comprises the cannula 66. The cannula 66 may be metal or a rigid plastic material. Advantageously, it may be bent for accommodating the oral situation. For instance the cannula may be bent such that it is oriented at an angle of 0 to 90°, preferably about 45° as shown in Fig. 7B, with regard to the tubular body 12 of the multiple-use dispensing device. Fig. 7B shows the system of Fig. 7A, whereby the single-use tubular dispensing tip 24 is screwed on the dispensing device 10. The elastic envelope 32 is designed in the form of a flexible tubular sheath with a first and second end, whereby said first end is provided at the proximal end of said tubular dispensing tip 24. It covers essentially the tubular body 12 of the dispensing device 10. This embodiment has the advantage that the handling of the dispensing device shielded at least at the distal portion thereof is facilitated when compared to the embodiment wherein the whole dispensing device is covered by the elastic envelope as illustrated in Fig. 5B and Fig. 6C. The elastic envelope 32 shown in Fig. 7B may also be tightened or clamped to the tubular body of the dispensing device 10.

The system of the present invention may be used as follows. The tubular body 12 of the multiple-use dispensing device may contain a dental material having gel consistency. During storage of the dental material the dispensing device is closed by the plug 40. The plug 40 may have a female connection section in case that the distal end of the tubular body 12 of the multiple-use dispensing device 10 has a male connection section protruding from the tubular body 12, such as tube 19. Alternatively, the plug 40 may have a male connection section in case that the distal end of the tubular body 12 of the multiple-use dispensing device 10 has a female connection section, such as the dispensing opening 18 itself or another aperture. The dispensing opening 18 itself or another part of the distal end 14 of the tubular body 12, such as the tube 19 or the above mentioned aperture may be connected to the plug 40 by engagement with a positive lock. Such a positive lock may be achieved by employing a thread, a bayonet locking, or a snapping connection. When dental material stored in the tubular body 12 of the dispensing device 10 shall be applied into the oral environment of a patient, the plug 40 is removed from the dispensing opening 18. After that, a single-use elastic envelope having an aperture 36 enabling a fluid-flow communication of dental material through said elastic envelope is placed on the dispensing opening 18. Then, the dispensing opening 18 is connected in fluid-flow communication to a single-use tubular dispensing tip 24. The single-use tubular dispensing tip 24 may have a female connection section 44 for receiving a male connection section of the distal end 14 of the tubular body 12, in case that the dispensing opening 18 or the distal end 14 of the tubular body 12 has a male connection section for insertion into a corresponding female connection section of the tubular dispensing tip 24. Alternatively, the single-use tubular dispensing tip 24 may have a male connection section for insertion into a female connection section of the distal end 14 of the tubular body 12, in case that the dispensing opening 18 or the distal end 14 of the tubular body 12 has a female connection section for receiving a corresponding male connection section of the tubular dispensing tip 24. According to another embodiment of the present invention, the single-use tubular dispensing tip 24 is connected to the dispensing device prior to placing the elastic envelope on the single-use tubular dispensing device (cf. Fig. 2). After that, the elastic envelope is placed completely over the whole dispensing device 10 or at least partially at least over the distal end of the dispensing device. Then, the dispensing device is ready for use, in order to dispense dental material into the oral environment of a patient, preferably directly onto an application site. The dispensing and application of dental material is achieved by moving the plunger 22 connected to the piston 20 into the tubular body 12 in which the dental material is contained. Thus, the dental material is forced through the dispensing opening 18 of the tubular body into the single-use dispensing tip 24. Further, the dental material is forced through the tubular dispensing tip 24 and through the dispensing orifice 30 which is pointed by the user to the application site in the oral environment of a patient. The dispensing device or at least a distal portion thereof is shielded by the elastic envelope from contamination by the oral environment. Preferably, the elastic envelope covers essentially the tubular body 12 of the dispensing device 10 as shown in Fig. 7B. After completion of dispensing dental material and applying it to the patient, the single-use elastic envelope and single-use tubular dispensing tip which are usually contaminated by saliva or other body fluids of the patient can be removed from the multiple-use dispensing device 10. Such removal can be conducted in a clean environment from a person different from the user who applied the dental material to the patient or by the user who applied the dental material to the patient after having disinfected or cleaned the hands contaminated generally by saliva or other body fluids of the patient. After removal of the elastic envelope 32 and the tubular dispensing tip, the dispensing opening of the dispensing device is closed again by a plug 40. The plug may be the same plug as the one which was used before for closing the dispensing opening 18 of the dispensing device. Alternatively, single-use plugs may be used. In this case a new single-use plug is used for closing the dispensing opening 18. The used single-use elastic envelope and single-use tubular dispensing tip are disposed.

Before, employing the multiple-use dispensing device for applying the dental material contained therein to another patient, the dispensing device or at least a distal part thereof may be shielded from the oral environment of another patient as described hereinbefore by removing the plug, and mounting a new single-use elastic envelope and a new single-use tubular dispensing tip onto the dispensing device.

According to a particular preferred embodiment of the present invention, the elastic envelope 32 of the system of the present invention is connected to the single-use tubular dispensing tip 24. In this case, the single-use tubular dispensing tip 24 is connected to the dispensing device 10 before the elastic envelope is placed on the dispensing device. More preferably, the elastic envelope 32 is folded or rolled up prior to use and prior to connecting the single-use tubular dispensing tip 24 to the distal end of the dispensing device 10. In case that such single-use tubular dispensing tip is used, connecting such tip to the dispensing device and covering the dispensing device by rolling the rolled-up elastic envelope down the tubular body may be conducted in a very fast manner, particularly, in case that such tip is connected to the dispensing device by a bayonet locking or a snapping connection.

## Claims

1. A system for dispensing a dental material having gel consistency, said system comprising:
(a) a multiple-use dispensing device (10) with
(a-1) a tubular body (12) having a distal end (14) and a proximal end (16), said body containing said dental material in an amount sufficient for application to more than one patient,
(a-2) a dispensing opening (18) provided at the distal end of said body,
(a-3) a movable piston (20), and
(a-4) a plunger (22) connected to said piston for dispensing said dental material through said dispensing opening;
(b) a plug (40) for closing said dispensing opening;
(c) a single-use tubular dispensing tip (24) having a proximal end (26) and a distal end (28), said dispensing tip being connectable in a fluid-flow communication to said dispensing opening after removal of said plug, and said distal end having a dispensing orifice (30) for dispensing said dental material into an oral environment; and
(d) a single-use elastic envelope (32) adapted for shielding said dispensing device or at least a distal portion thereof from contamination by the oral environment.

2. The system according to claim 1, whereby said elastic envelope is connected to said dispensing tip.

3. The system according to claim 1 or 2, whereby the elastic envelope is a flexible tubular sheath with a first and second end, whereby said first end is provided at the proximal end of said tubular dispensing tip.

4. The system according to claim 3, whereby the second end of said sheath can be tightened, preferably by a cord or ribbon.

5. The system according to claim 3 or 4, whereby said elastic envelope is folded up or rolled up prior to use.

6. The system according to one of claims 1 to 5, whereby the contact surfaces of said dispensing tip and said body are adapted for a snug fitting.

7. The system according to one of claims 1 to 6, whereby said tubular dispensing tip can be bent for accommodating the oral situation.

8. The system according to one of claims 1 to 7, whereby the tubular dispensing tip is adapted for engaging with said dispensing device with a positive lock.

9. The system according to claim 8, whereby said positive lock is a thread, a bayonet locking, or a snapping connection.
